# EUROPÄISCHE PATENTSCHRIFT

(11) **EP 0 450 121 B1**
(45) Veröffentlichungstag und Bekanntmachung des Hinweises auf die Patenterteilung: **23.11.1994**
(21) Anmeldenummer: 90106470.9
(22) Anmeldetag: 04.04.1990
(51) Int. Cl.: A61F 2/38

(54) **Schienbeinteil einer Kniegelenk- Endoprothese**
Tibial component of a knee joint endoprosthesis
Partie de tibia d'une endoprothèse pour l'articulation du genou

(43) Veröffentlichungstag der Anmeldung: 09.10.1991
(73) Patentinhaber: S + G IMPLANTS GMBH, D-23556 Lübeck (DE)
(72) Erfinder: Henssge, Ernst Joachim, Prof.Dr.Med., D-2400 Lübeck (DE); Köller, Wolfgang, Dr., D-2400 Lübeck 16 (DE); Scholz, Jörg, Dr.Med., D-1000 Berlin 31 (DE); Dufek, Pavel, Dr. med., D-2400 Lübeck (DE)
(74) Vertreter: Eisenführ, Speiser & Partner

(56) Entgegenhaltungen:
- EP-A- 0 333 642
- WO-A-85/02535
- DE-A- 2 851 598
- DE-A- 3 006 178
- DE-U- 8 713 071

## Beschreibung

Die Erfindung betrifft ein Schienbeinteil einer Kniegelenk-Endoprothese mit einem in die Markhöhle des Schienbeins einzubringenden Verankerungsstiel, der einendseitig mit einer Schienbeinkopfabschlußplatte zur Befestigung der Gelenkteile der Prothese versehen ist, wobei der Verankerungsstiel aus einer zylindrischen, in eine zylindrische Schlitzbohrung der Tibia einführbaren Hülse mit einem den Spongiosa-Kern der Schlitzbohrung aufnehmenden Innenraum besteht und im rechten Winkel zu der parallel zur waagerechten oder leicht geneigten Knochenresektionsfläche verlaufenden Abschlußplatte stehend ist, wobei die Hülse aus einem metallischen oder einem anderen geeigneten körperverträglichen Werkstoff besteht und in ihrem zylindrischen Mantel eine Anzahl von Durchbrechungen aufweist.

Aus der WO-A-8502535 ist ein derartiges Schienbeinteil bekannt, welches als Verankerungsstiel eine zylindrische Hülse mit einer Anzahl von Durchbrechungen im Hülsenmantel zeigt. Bei diesem bekannten Schienbeinteil wird das Spongiosagewebe des Schienbeinknochens nach der Implantation der Prothese durch die Durchbrechungen des Hülsenmantels hindurchwachsen und dadurch die Prothese fest im Knochen verankern. Aufgrund des festen Verbundes zwischen dem prothetischen Schienbeinteil und dem Knochen ist es jedoch oftmals problematisch, das prothetische Schienbeinteil nach längerer Benutzung wieder aus dem Knochen herauszunehmen, falls dies erforderlich sein sollte.

Aufgabe der Erfindung ist es daher, ein Schienbeinteil der eingangs genannten Art derart weiterzubilden, daß die Heraustrennung des Schienbeinteils aus dem Knochengewebe stets in einfacher Weise möglich ist.

Diese Aufgabe wird bei dem Schienbeinteil der eingangs genannten Art erfindungsgemäß dadurch gelöst, daß die die Durchbrechungen begrenzenden Ränder der Hülse messerklingenartig ausgebildet sind, daß die Hülse in ihrem Innenraum einen zweiten, an der Hülseninnenwandfläche anliegenden hülsenförmigen Formkörper aufweist, der mit einer Anzahl von mit den Durchbrechungen der Hülse sich deckenden Durchbrechungen versehen ist, wobei die die einzelnen Durchbrechungen des hülsenförmigen Formkörpers begrenzenden Ränder messerklingenartig ausgebildet sind, daß der innere hülsenförmige Formkörper mit seinem oberen Ende bis in den Bereich einer mittig in der Abschlußplatte ausgebildeten Durchbrechung verlängert ist und an seinem oberen Ende innenwandseitig mit Eingriffnocken für ein auf den hülsenförmigen Formkörper ansetzbaren und mit diesem in Wirkverbindung bringbaren Werkzeug zwecks Verdrehen des hülsenförmigen Formkörpers um seine mittlere Längsachse versehen ist.

Die Vorteile der Erfindung liegen insbesondere darin, daß die Durchbrechungen im Hülsenmantel und im hülsenförmigen Formkörper beim Implantieren in Register gebracht werden können, so daß das Knochengewebe in den Innenraum des Formkörpers hineinwachsen und das prothetische Schienbeinteil fest verankern kann. Wenn dann nach längerer Nutzung eine Explantation des prothetischen Schienbeinteiles notwendig sein sollte, so wird lediglich durch Verdrehen des hülsenförmigen Formkörpers das durch die Durchbrechungen hindurchgetretene Knochengewebe durchtrennt, und diese Durchtrennung wird durch die klingenartige Ausgestaltung der Randbereiche der Durchbrechungen erleichtert. Auf diese Weise läßt sich das prothetische Schienbeinteil sauber aus dem Knochen heraustrennen, ohne daß es zu unerwünschten großräumigen Zerstörungen des Knochengewebes kommt.

Weitere vorteilhafte Ausgestaltungen der Erfindung sind in den Unteransprüchen gekennzeichnet.

Ein Ausführungsbeispiel der Erfindung wird nachstehend anhand der Zeichnung näher erläutert. Es zeigen
Fig. 1 teils in Ansicht, teils in einem senkrechten Schnitt einen in das Schienbein implantierten Stiel des Schienbeinteils einer Kniegelenk-Endoprothese,
Fig. 2 teils in Ansicht, teils in einem senkrechten Schnitt ein Schienbein mit einer zylindrischen Schlitzbohrung zur Aufnahme des hülsenartig ausgebildeten Verankerungsstiels,
Fig. 3 in medial-lateral-Ansicht das Schienbein mit implantiertem Verankerungsstiel,
Fig. 4 in einer schaubildlichen Ansicht den Verankerungsstiel mit seiner oberen Abschlußplatte,
Fig. 5 in einem senkrechten Schnitt ein erfindungsgemäßen Schienbeinteil einer Kniegelenk-Endoprothese.

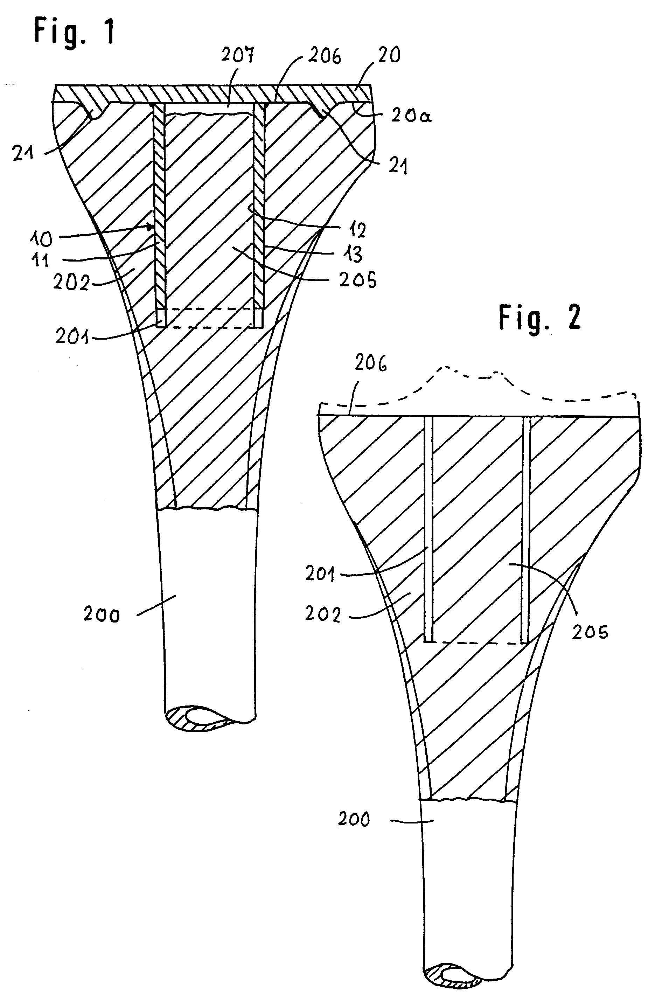

In Fig. 1,3 und 4 ist mit 10 der Verankerungsstiel eines Schienbeinteils einer Kniegelenk-Endoprothese bezeichnet, der in die Markhöhle des Schienbeins einzubringen ist. Dieser Verankerungsstiel 10 besteht aus einer zylindrischen Hülse 11, aus einem metallischen oder einem anderen geeigneten, körperverträglichen Werkstoff. Der Hülseninnenraum ist mit 12 und der die äußere Wand bildende Hülsenmantel mit 13 bezeichnet.

An einem ihrer beiden Enden trägt die Hülse 11 eine Abschlußplatte 20, deren der Hülse 11 zugekehrte Wandfläche 20a eine Anzahl von Knochenarretierungsnasen oder -zapfen 21 trägt. Eine Verdrehsicherheit des Verankerungsstiels 10 nach erfolgter Implantation wird bereits erreicht, wenn die Abschlußplatte 20 an ihrer der Knochenresektionsfläche 206 zugekehrten Wandfläche 20a mit einer Knochenarretierungsnase oder -zapfen 21 versehen ist.

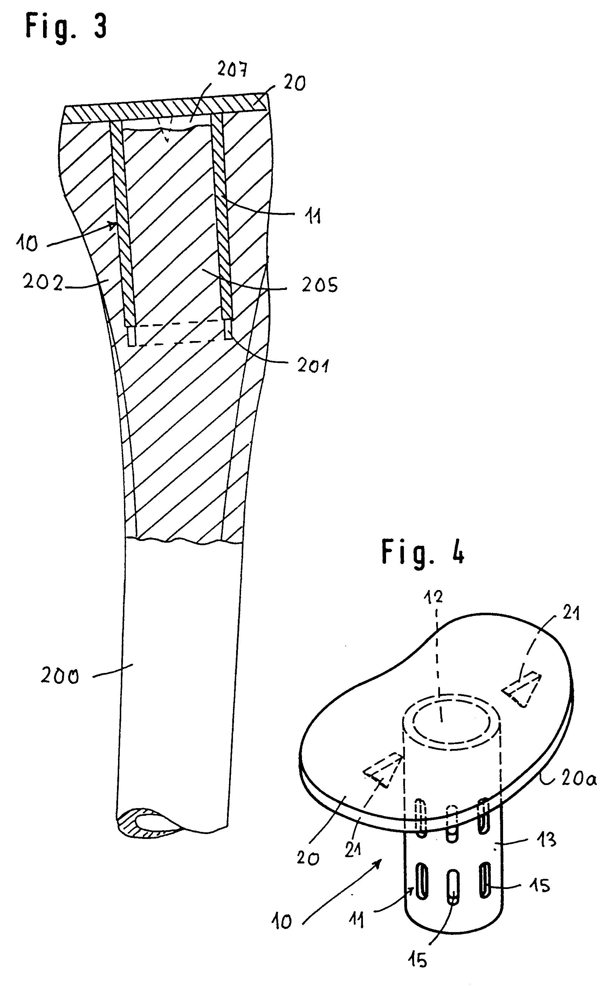

Die Implantation des Verankerungsstiels 10 erfolgt in einer im Schienbein 200 ausgebildeten zylindrischen Schlitzbohrung 201 (Fig.2). Nach Anbringen dieser zylindrischen Schlitzbohrung 201 verbleibt ein mittiger Kern 205 der Spongiosa 202. Der Durchmesser dieser Schlitzbohrung 201 und deren Abmessungen sind zur Hülse 11 des Verankerungsstiels 10 derart, daß nach dem Einbringen bzw. Eintreiben der Hülse 11 in die Schlitzbohrung 201 die Hülse 11 unter Paßsitz in der Schlitzbohrung 201 gehalten ist. Vorzugsweise weist der durch die Schlitzbohrung 201 ausgebildete Wandteil eine Breite auf, die etwas geringer ist als die Materialstärke der Hülse 11, so daß nach dem Implantieren des Verankerungsstiels 10 in der Spongiosa 202 und in der Markhöhle des Schienbeins 200 zwischen dem Hülsenmantel 13 und dem Knochenmaterial kein Spielraum erhalten wird, der durch eine Zementmasse ausgefüllt bzw. verschlossen werden muß. Vorzugsweise ist die Länge der Schlitzbohrung 201 etwas größer bemessen als die Länge der Hülse 11 des Verankerungsstiels 10, so daß nach erfolgter Implantation die Abschlußplatte 20 mit ihrer Wandfläche 20a vollflächig auf der Knochenresektionsfläche 206 aufliegt.

Der durch die Schlitzbohrung 201 erhaltene Spongiosa-Kern 205 füllt nach erfolgter Implantation den Innenraum 12 der Hülse aus. Dabei kann sich der Spongiosa-Kern 205 über die gesamte Hülsenlänge erstrecken und den gesamten Hülseninnenraum 12 ausfüllen. Es besteht darüber hinaus jedoch auch die Möglichkeit, im Innenraum 12 der Hülse 11 einen oberen spongiosafreien Raum 207 auszubilden, so daß der im Hülseninnenraum 12 liegende Spongiosa-Kern 205 eine gegenüber der Länge der Hülse 11 kürzere Länge aufweist. Die Ausbildung eines oberen spongiosafreien Raumes 207 im Innenraum 12 der Hülse 11 nach erfolgter Implantation des Verankerungsstiels 10 in das Schienbein 200 sichert die vollflächige Anlage der Abschlußplatte 20 auf der Knochenresektionsfläche 206.

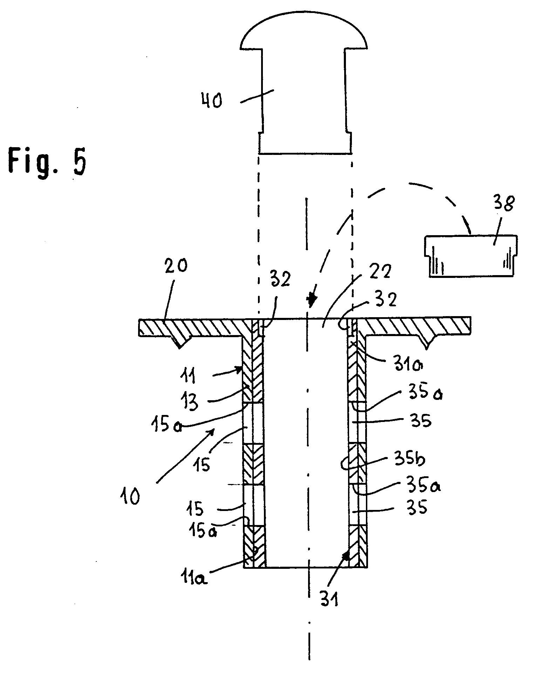

Um die Ernährung des Knochens bzw. der Spongiosa im Innenraum der Hülse 11 nach erfolgter Implantation des Verankerungsstiels 10 zu gewährleisten, weist der Mantel 13 der Hülse 11 eine Anzahl von gleichmäßig oder ungleichmäßig angeordneten Durchbrechungen 15 auf (Fig.4).

Gemäß der Erfindung sind die die einzelnen in dem zylindrischen Mantel 13 der Hülse 11 ausgebildeten Durchbrechungen 15 begrenzenden Ränder 15a messerklingenartig mit der Wirkung eines Schneidmessers ausgebildet, vgl. Fig. 5. Die Hülse 11 des Verankerungsstiels 10 weist in ihrem Innenraum 12 einen weiteren, an der Hülseninnenwandfläche 11a anliegenden hülsenförmigen Formkörper 31 auf,der aus einem metallischen oder einem anderen geeigneten, körperverträglichen Werkstoff besteht. Dieser hülsenförmige Formkörper 31 ist mit einer Anzahl von mit den Durchbrechungen 15 der Hülse 11 sich deckenden Durchbrechungen 35 versehen, wobei die die einzelnen Durchbrechungen 35 des hülsenförmigen Formkörpers 31 begrenzenden Ränder 35a ebenfalls messerklingenartig mit der Wirkung eines Schneidmessers ausgebildet sind. Die Größe und die Formgebung dieser Durchbrechungen 35 entsprechen dabei denen der Durchbrechungen 15 im Mantel 13 der Hülse 11. Die Implantation eines derart ausgebildeten Verankerungsstiels 10 erfolgt ebenfalls in einer im Schienbein 200 ausgebildeten zylindrischen Schlitzbohrung 201, die so bemessen ist, daß die Hülse 11 mit ihrem innenliegenden hülsenförmigen Formkörper 31 in diese Schlitzbohrung 201 einsetzbar ist. Im implantierten Zustand nimmt der hülsenförmige Formkörper 31 dann zur Hülse 11 eine Stellung ein, bei der sich die Durchbrechungen 15 der Hülse 11 mit den Durchbrechungen 35 des hülsenförmigen Formkörpers 31 decken. Ein derart ausgebildeter und implantierter Verankerungsstiel 10 gewährleistet eine ausreichende Ernährung des Knochens, da die sich miteinander deckenden Durchbrechungen 15,35 den Durchtritt von Blutgefäßen ermöglichen, so daß der Knochen im Innenraum 12 der Hülse 11 ausreichend ernährt werden kann. Hinzu kommt, daß ein Verwachsen des Knochens mit demjenigen Knochenteil durch diese Durchbrechungen möglich ist, der sich im Innenraum 12 der Hülse 11 befindet.

Der innere hülsenförmige Formkörper 31 ist mit seinem oberen Ende 31a bis in den Bereich einer mittig in der Abschlußplatte 20 ausgebildeten Durchbrechung 22 verlängert. Des weiteren weist der Formkörper 31 an seinem oberen Ende in seiner Innenwandfläche 35a ausgebildete Eingriffnocken 32 für ein auf den hülsenförmigen Formkörper 31 ansetzbaren und mit diesem in Wirkverbindung bringbaren Werkzeug 40 auf, um ein Verdrehen des hülsenförmigen Formkörpers 31 um seine mittlere Längsachse zu ermöglichen. Als Eingriffwerkzeug 40 kann ein wie in Fig. 5 dargestellter Schlüssel Verwendung finden. Im implantierten Zustand ist die in der oberen Abschlußplatte 20 vorgesehene Durchbohrung 22 vermittels einer Verschlußklappe 38 verschlossen. Diese Verdrehmöglichkeit des hülsenförmigen Formkörpers 31 ist insofern von Vorteil, als dadurch die Möglichkeit gegeben ist, einen implantierten Verankerungsstiel 10 mühelos aus dem Schienbein 200 wieder herauszunehmen, falls dies erforderlich sein sollte. Lediglich durch Verdrehen des hülsenförmigen Formkörpers 31 werden die durch die Durchbrechungen 15,35 hindurchgetretenen Blutgefäße und die durch die Durchbrechungen hindurchgewachsene Knochensubstanz zertrennt, wobei diese Zertrennung durch die messerklingenartige Ausgestaltung der Randbereiche der Durchbrechungen 15,35 unterstützt wird.

Die Hülse 11 des Verankerungsstiels 10 weist einen kreisförmigen Querschnitt auf.

## Patentansprüche

1. Schienbeinteil einer Kniegelenk-Endoprothese mit einem in die Markhöhle des Schienbeins einzubringenden Verankerungsstiel, der einendseitig mit einer Schienbeinkopfabschlußplatte zur Befestigung der Gelenkteile der Prothese versehen ist, wobei der Verankerungsstiel (10) aus einer zylindrischen, in eine zylindrische Schlitzbohrung (201) der Tibia einführbaren Hülse (11) mit einem den Spongiosa-Kern (205) der Schlitzbohrung (201) aufnehmenden Innenraum (12) besteht und im rechten Winkel zu der parallel zur waagerechten oder leicht geneigten Knochenresektionsfläche (206) verlaufenden Abschlußplatte (20) stehend ist, wobei die Hülse (11) aus einem metallischen oder einem anderen geeigneten körperverträglichen Werkstoff besteht und in ihrem zylindrischen Mantel (13) eine Anzahl von Durchbrechungen (15) aufweist,
dadurch gekennzeichnet, daß die die Durchbrechungen (15) begrenzenden Ränder (15a) der Hülse (11) messerklingenartig ausgebildet sind, daß die Hülse (11) in ihrem Innenraum (12) einen zweiten, an der Hülseninnenwandfläche (11a) anliegenden hülsenförmigen Formkörper (31) aufweist, der mit einer Anzahl von mit den Durchbrechungen (15) der Hülse (11) sich deckenden Durchbrechungen (35) versehen ist, wobei die die einzelnen Durchbrechungen (35) des hülsenförmigen Formkörpers (31) begrenzenden Ränder (35a) messerklingenartig ausgebildet sind, daß der innere hülsenförmige Formkörper (31) mit seinem oberen Ende (31a) bis in den Bereich einer mittig in der Abschlußplatte (20) ausgebildeten Durchbrechung (22) verlängert ist und an seinem oberen Ende innenwandseitig (35a) mit Eingriffnocken (32) für ein auf den hülsenförmigen Formkörper (31) ansetzbaren und mit diesem in Wirkverbindung bringbaren Werkzeug (40) zwecks Verdrehen des hülsenförmigen Formkörpers (31) um seine mittlere Längsachse versehen ist.

2. Schienbeinteil nach Anspruch 1,
dadurch gekennzeichnet, daß die mittige Durchbrechung (22) in der Abschlußplatte (20) mittels einer Verschlußkappe (38) verschließbar ist.

3. Schienbeinteil nach Anspruch 1 oder 2,
dadurch gekennzeichnet, daß die Hülse (11) einen kreisförmigen Querschnitt aufweist.

## Claims

1. The tibia part of a kneejoint endoprosthesis, which for introducing it into the medullary cavity in the tibia has an anchoring-stem provided at one end with a tibia-head terminal-plate for fastening the hinge parts of the prosthesis, where the anchoring-stem (10) consists of a cylindrical sleeve (11) which may be introduced into a cylindrical slit (201) bored in the tibia and receive in the apace (12) inside it the spongy care (205) of the bored slit (201), the sleeve (11) standing at right angles to the terminal plate (20) which runs in parallel with the horizontal or slightly inclined bone resection face (206), and consisting of a metallic or other suitable bodily-compatible material and exhibiting in its cylindrical shell (13) a number of openings (15),
characterized in that the edges (15a) bounding the openings (15) in the sleeve (11) are made like knifeblades, that in the space (12) inside it the sleeve (11) exhibits a second body (31) shaped in the form of a sleeve which rests against the face (11a) of the wall inside the sleeve and is provided with a number of openings (35) which coincide with the openings (15) in the sleeve (11), the edges (35a) bounding the individual openings (35) in the sleeve-shaped body (31) being made like knifeblades, that the sleeve-shaped inner body (31) is prolonged at its top end (31a) up into the region of an opening (22) made in the middle of the terminal plate (20) and is provided at its top end next the inner wall (35a) with engagement dogs (32) for a tool (40) which may be mounted on the sleeve-shaped body (31) and brought into operative connection with it for the purpose of twisting the sleeve-shaped body (31) about its longitudinal centreline.

2. a tibia part as in Claim 1, characterized in that
the opening (22) in the centre of the terminal plate (20) nay be closed by means of a closure cap (38).

3. a tibia part as in Claim 1 or 2. characterized in that
the sleeve (11) exhibits a circular cross-section.

## Revendications

1. Partie tibiale d'une endoprothèse de l'articulation du genou, comportant une tige d'ancrage destinée à être insérée dans le canal médullaire du tibia, et qui est pourvue à une extrémité, d'une plaque terminale d'épiphyse tibiale pour la fixation des parties d'articulation de la prothèse, la tige d'ancrage (10) étant constituée d'une douille cylindrique (11) qui est à insérer dans un alésage annulaire cylindrique (201) du tibia et possède un espace intérieur (12) recevant le tissu spongieux central (205) de l'alésage annulaire (201), la tige d'ancrage étant agencée de manière à être perpendiculaire à la plaque terminale (20) s'étendant parallèlement à la surface de résection de l'os (206) horizontale ou légèrement inclinée, la douille (11) étant réalisée en un matériau métallique ou en un autre matériau approprié, compatible avec les tissus du corps humain, et comportant dans son enveloppe cylindrique (13), un certain nombre d'ouvertures de passage (15),
caractérisée en ce que les bords (15a) délimitant les ouvertures de passage (15) de la douille (11), présentent une configuration en forme de lame de coupe, en ce que la douille (11) comporte dans son espace intérieur (12), un second corps de forme (31) en forme de douille qui s'applique contre la paroi intérieure (11a) de la douille, et qui comporte un certain nombre d'ouvertures de passage (35) en regard des ouvertures de passage (15) de la douille (11), les bords (35a) délimitant les ouvertures de passage (35) individuelles du corps de forme (31) en forme de douille, présentant une configuration en forme de lame de coupe, en ce que le corps de forme intérieur (31) en forme de douille se prolonge, avec son extrémité supérieure (31a), jusque dans la zone d'un passage (22) réalisé de manière centrale dans la plaque terminale (20), et est pourvu à son extrémité supérieure, sur sa paroi intérieure, de crans d'entraînement (32) destiné à un outil (40) pouvant être appliqué sur le corps de forme (31) en forme de douille et amené en liaison fonctionnelle avec celui-ci, en vue de faire tourner le corps de forme (31) en forme de douille, autour de son axe central longitudinal.

2. Partie tibiale selon la revendication 1, caractérisée en ce que le passage centrale (22) dans la plaque terminale (20) peut être obturé par un capuchon de fermeture (38).

3. Partie tibiale selon la revendication 1 ou 2, caractérisée en ce que la douille (11) présente une section transversale circulaire.
